**Europäisches Patentamt**

(19) **European Patent Office**

**Office européen des brevets**

(11) Veröffentlichungsnummer: **0 045 914**

**A1**

(12) # EUROPÄISCHE PATENTANMELDUNG

(21) Anmeldenummer: **81106033.4**

(22) Anmeldetag: **31.07.81**

(51) Int. Cl.³: **A 61 B 5/10**

(30) Priorität: **11.08.80 US 176696**

(43) Veröffentlichungstag der Anmeldung:
**17.02.82 Patentblatt 82/7**

(84) Benannte Vertragsstaaten:
**AT BE CH DE FR GB IT LI NL SE**

(71) Anmelder: **SIEMENS AKTIENGESELLSCHAFT Berlin
und München
Postfach 22 02 61
D-8000 München 22(DE)**

(72) Erfinder: **Rüll, Hartwig, Dr.
Balduin-Helm-Strasse 39
D-8080 Fürstenfeldbruck(DE)**

(54) **Eingabesensoreinheit für ein System zur Überprüfung eines Fingerabdrucks.**

(57) Es wird eine Eingabesensoreinheit für ein System zur Überprüfung eines Fingerabdrucks beschrieben. Eine Sensorplatte (3), die einem Fingerbett (1) gegenüberliegt, besteht aus einem transparenten Polymer, welches derart elastisch ist, daß es ein latentes topografisches Relief eines Fingermusters ausbildet. Ein abtastender Lichtstrahl (40) wird durch eine Randfläche (34) der Sensorplatte (3) in diese eingekoppelt. Ein aus einer zweiten planaren Fläche (32) der Sensorplatte austretender Lichtstrahl (5, 5') wird einem lichtempfindlichen Bereich (71) eines elektrooptischen Sensorfeldes (7) zugeführt. Im Strahlengang des austretenden Lichtstrahls (5, 5') ist eine optische Linse (6) angeordnet, welche den Strahl auf den lichtempfindlichen Bereich (71) fokussiert und ein optisches Filter (8) blendet das im austretenden Lichtstrahl enthaltene direkte Licht (5) aus.

FIG. 1

SIEMENS AKTIENGESELLSCHAFT      Unser Zeichen
Berlin und München              VPA 80 P 8230

Eingabesensoreinheit für ein System zur Überprüfung eines Fingerabdrucks

Die Erfindung bezieht sich auf ein System zur Überprüfung eines Fingerabdrucks gemäß dem Oberbegriff des Patentanspruchs 1.

Systeme zur Identifizierung von Fingerabdrücken, welche in Wirklichkeit nicht den Abdruck eines Fingers identifizieren, sondern den auf eine Kontaktfläche gedrückten Finger selbst, sind bekannt. Ein derartiges Gerät zur Identifizierung eines Fingers geht aus der US-PS 4 053 228 hervor. Dieses Gerät weist als Kontaktfläche für den Finger eine transparente Glasplatte auf. Eine räumlich kohärentes Licht erzeugende Lichtquelle sendet einen abfragenden Lichtstrahl aus, der durch die Vorderfläche der Glasplatte geführt wird. Wenn ein Finger auf diese Fläche aufgelegt wird, wird der Lichtstrahl aufgrund von optischen Diskontinuitäten zwischen der Glasplatte und dem Finger teilweise an der Rückfläche der Glasplatte reflektiert. Es gibt zwei verschiedene Arten von Diskontinuitäten: Eine Diskontinuität findet zwischen der Glasplatte und der unter den Rillen des Fingers liegenden Luft statt. Eine andere Diskontinuität erscheint zwischen der Glasplatte und den Rippen oder Erhöhungen des Fingers. Diese Diskontinuitäten bewirken, daß unterschiedliche Lichtmengen reflektiert werden und daß ein die Fingerabdruckinformation tragender, reflektierter Signalstrahl auf diese Weise erzeugt wird. Dieser modulierte, reflektierte Signalstrahl wird mit einem Hologramm desselben Fingerabdrucks korreliert, so daß eine Identifizierung geliefert wird.

Ed 1 Sti/10.7.81

-2- VPA 80 P 8230

Es ist darauf hinzuweisen, daß der Informationsgehalt des reflektierten Lichtstrahls in hohem Maße von den Unterschieden dieser Diskontinuitäten abhängt. Der reflektierte Lichtstrahl kann zwischen den Erhebungen und Vertiefungen des Fingerabdrucks schwache Kontraste aufweisen, wenn der Finger während des Kontaktes mit der Glasfläche nicht absolut stillgehalten wird. Des weiteren können unterschiedliche, während des Vergleichsprozesses ausgeübte Druckkräfte Verschwommenheiten oder Verzerrungen im Fingerabdruck zur Folge haben.

Ein anderes System zur Identifizierung von Fingerabdrücken geht aus der US-PS 4 120 585 hervor. Dieses System bezieht sich auf ein nachgiebiges optisches Prisma in einem Endgerät zum Abtasten eines Fingerabdrucks, bei dem eine Fläche des Prismas von einem Finger berührt wird. Im Grunde umfaßt das System ein optisches Element, welches durch einen Fingerdruck bis zu einem gewissen Umfang deformierbar ist, wodurch die Kontaktfläche mit einem Finger erhöht wird. Das optische Element gewinnt seine ursprüngliche Form wieder, wenn der Druck fortgenommen wird. Bei richtigem Berührungsdruck des Fingers wird ein abfühlender Lichtstrahl teilweise auf eine fotoempfindliche Vorrichtung reflektiert, die dadurch aktiviert wird. Diese fotoempfindliche Vorrichtung aktiviert ihrerseits das Endgerät. Der einzige Grund für die Nachgiebigkeit des Prismas liegt darin, eine positive Voraussetzung für einen hinreichenden Fingerdruck zur Aktivierung des Systems zu erhalten. Obwohl das bekannte nachgiebige, optische Prisma während des Betriebes eine geriffelte Oberfläche ausweist, die als Spiegel wirkt, weist es dennoch ein sehr kleines topografisches Relief auf. Entsprechend der durch die Patentschrift gegebenen Lehre wird angenommen, daß das Prisma nicht arbeiten würde, wenn es
in die Vertiefungen des Fingers eindringen und diese
ausfüllen könnte.

Aus der US-PS 3 982 836 geht ein Verfahren und eine
Einrichtung zum Verstärken von Abdrücken für eine
direkten Vergleich hervor. Das Verfahren verwendet
zur Gewinnung eines Druckmusters ein transparentes,
druckempfindliches Gel. Während der Finger auf die
klare ebene Oberfläche des transparenten, druckempfindlichen Gels gepreßt wird, das in Form eines
Filmes oder Bandes bzw. Streifens ausgebildet ist,
bewirkt der Kontakt mit den Erhebungen und Vertiefungen des Fingers eine relativ starke Störung der
optischen Qualität der Oberfläche. Das System umfaßt
ein langgestrecktes, druckempfindliches Band und
Einrichtungen, mit denen es vorwärts bewegbar ist,
so daß sukzessive klare Abschnitte dargeboten und
eine Anzahl von Abdruckbildern in einer einzigen
Reihe gemacht und erhalten werden können. Ein in
der Oberfläche des Bandes ausgebildeter Abdruck
eines Objekts stellt folglich eine lang haltbare
Aufzeichnung dar.  Es wird angenommen, daß bei geeigneter Wahl des druckempfindlichen Materials eine Aufzeichnung erhalten werden kann, die als im wesentlichen
permanent bezeichnet werden kann.

Das Gel wird von einem Strahl kollimierten und polarisierten Lichts durchstrahlt. Die Züge des in die Oberfläche des Gels eingedrückten Musters streuen Licht
aus dem Strahlengang heraus. Der Strahl wird dann
auf ein zweites Polarisationselement gerichtet, welches
so orientiert ist, daß es die gestreuten Lichtstrahlen
herausfiltert und dadurch ein Abdruckbild hohen Kontrastes erzeugt, das für die direkte Beobachtung oder eine automatisierte Weiterverarbeitung geeignet ist.

Die verschiedenen Systeme zur Abtastung von Fingerabdrücken und die entsprechenden Techniken erfüllen nicht alle praktischen Erfordernisse einer wirklich einfachen und ausführbaren Sensorvorrichtung für ein System zur Überprüfung eines Fingerabdrucks, die bei der Erzeugung eines zum Abfragen von Fingerabdrücken dienenden Geräts höchster Qualität und Kontrastes. erfüllt sein müssen, damit unverfälschte, für Speicher- oder Vergleichszwecke dienende Information erhalten wird.

Die Aufgabe der vorliegenden Erfindung besteht deshalb darin, eine verbesserte Eingabesensoreinheit für ein System zur Überprüfung eines Fingerabdrucks zu schaffen.

Diese Aufgabe wird durch die im kennzeichnenden Teil des Patentanspruchs 1 angegebenen Merkmale gelöst.

Bevorzugte und vorteilhafte Ausführungsformen der Erfindung gehen aus den Unteransprüchen 2 bis 13 hervor.

Durch die Erfindung ist eine Eingabesensoreinheit für ein System zur Überprüfung eines Fingerabdrucks geschaffen worden, welche optische Einrichtungen aufweist, mit denen ein Fingerabdruck hoch aufgelöst werden kann und Einzelheiten des Fingerabdrucks besser erkannt werden können, unabhängig von Unterschieden bei individuellen Reaktionen auf Gebrauchsanweisungen oder den tatsächlichen vorkommenden Fingern, deren Abdrücke nachzuprüfen sind.

Durch die Erfindung ist auch eine Eingabesensoreinheit für ein System zur Überprüfung eines Fingerabdrucks geschaffen worden, deren Abtastergebnisse unabhängig von Rückständen von Fingerabdrücken sind, die beispielsweise auf Abtastplatten oder Prismen aus Glas zurückbleiben, und weitere Abtastproben zerstören können, wenn sie nicht entfernt werden.

Ein weiterer Vorteil der Erfindung liegt darin, daß eine farbfreie, trocken durchführbare Technik zur Überprüfung eines Fingerabdrucks geschaffen worden ist, die in Werkschutz- und Sicherheitssystemen leicht verwendbar ist.

Durch die vorliegende Erfindung ist somit eine Eingabesensoreinheit für ein System zur Überprüfung eines Fingerabdrucks geschaffen worden, welches ein Fingerbett mit einer Öffnung zur Aufnahme eines Fingers aufweist, dessen Abdruck zu überprüfen ist. Beim Fingerbett und parallel dazu ist eine mit einer ersten und einer zweiten großen planaren Fläche und mit Randflächen versehene Sensorplatte angeordnet, welche sich über die ganze Öffnung erstreckt. Die Sensorplatte ist aus einem transparenten Polymer zusammengesetzt, welches elastisch ist, so daß es ein latentes topografisches Relief eines Fingermusters ausbildet, während es dem Finger ausgesetzt ist.

Zum Erzeugen eines durch eine Randfläche in die Sensorplatte eindringenden, abtastenden Lichtstrahles ist eine Beleuchtungseinrichtung vorgesehen, und der Lichtstrahl wird in der Sensorplatte an den planen parallelen Flächen so lange total reflektiert wie die erste planare Fläche nicht von einem Finger berührt wird. Lokale Störungen oder Verformungen der ersten planaren Fläche in Gegenwart des Fingers haben zur Folge, daß reflektierte Lichtstrahlen, welche die zweite planare Fläche auf der Gegenseite des Fingerbetts in Einfallswinkeln treffen, die kleiner als der Totalreflexionswinkel sind. Dies resultiert in einem austretenden Lichtstrahl, der mit der die Fingerabdruckstruktur enthaltenen Information moduliert ist.

Ein elektrooptisches Sensorfeld mit einem lichtempfindlichen Bereich ist dem austretenden Lichtstrahl ausgesetzt, um den modulierten austretenden Lichtstrahl in elektrische Signale umzuwandeln, welche die Information des Fingerabdrucks enthalten. Zur Verbesserung der Qualität des auf den lichtempfindlichen Bereich des elektrooptischen Sensorfeldes geworfenen Bildes sind eine der Sensorplatte gegenüberliegende Linse und ein zwischen der Linse und dem elektrooptischen Sensorfeld angeordnetes optisches Filter vorgesehen, die so einjustiert sind, daß ein Teil des austretenden Lichtstrahls, der als direktes Licht zu bezeichnen ist und der senkrecht aus der zweiten planaren Fläche der Sensorplatte austritt, aus dem austretenden Lichtstrahl ausgeblendet wird.

Diese Anordnung weist mehrere Vorteile auf. Bekannte Eingabesensoreinheiten machen oft Gebrauch von einer klaren Sensorplatte oder einem Sensorprisma, die aus Glas gefertigt sind, und es wird ein aus Hautöl- oder Talgabscheidungen geformtes Fingermuster ausgewertet. Diese Abscheidungen hängen von einer Anzahl von Faktoren, beispielsweise der Temperatur, dem emotionalen Zustand und dem Alter einer zu überprüfenden Person ab. Darüber hinaus wird die praktische Brauchbarkeit bekannter Eingabesensoren dadurch eingeschränkt, daß der Finger, um Abdrücke hoher Qualität zu erhalten, während des Aufpressens auf die Sensorfläche stillgehalten werden muß. Die vorliegende Erfindung überwindet diese Einschränkungen, weil die planare Fläche der Sensorplatte, die einem Finger ausgesetzt wird, ein latentes, echtes topografisches Relief des Fingermusters bildet, welches durch Talgabscheidungen nicht zerstört oder gestört wird. Dieses latente Relief wird optisch abgetastet, was durch ein unkompliziertes optisches System ausgeführt werden kann, das die Eigenschaften der inneren Totalreflexion in der planparalleln Sensorplatte ausnutzt.

Die den abtastenden Lichtstrahl erzeugende Lichtquelle kann eine kohärente oder inkohärente Quelle sein. Gemäß bevorzugter Ausführungsformen der Erfindung werden röhrenförmige Lichtquellen, beispielsweise fluoriszierende Röhren,zur Erzeugung des abtastenden Lichtstrahls verwendet. Lichtquellen können so angeordnet werden, daß sie einer oder mehreren oder allen Randflächen der Sensorplatte gegenüberliegen, wobei im letztgenannten Fall hinsichtlich einer diffusen Verteilung des reflektierten Lichts in einer relativ kleinen Sensorplatte beste Ergebnisse erhalten werden. Unterschiedliche mechanische Anordnungen können erhalten werden, indem zum Einkoppeln des abtastenden Lichtstrahls in die Sensorplatte zylindrische Linsen oder Totalreflexionsprismen verwendet werden.

Die Sensorplatte selbst kann aus einer Mischung aus elastischen Polymeren zusammengesetzt sein, derart, daß ihr Brechungsindex in Abhängigkeit von der Eindringtiefe senkrecht zu ihrer großen planaren Fläche variiert. Die Variation dieses Brechungsindex kann auf die Eindringtiefe eines Fingers begrenzt bleiben oder über die ganze Stärke der Sensorplatte unbegrenzt sein.

Die Qualität des ausgegebenen Bildes kann des weiteren durch Fokussierung und räumliche Filterung in herkömmlichen optischen Einrichtungen, beispielsweise durch eine Schneide oder ein optisches Bandpaßfilter, verbessert werden. Eine optische Linse fokussiert den modulierten, austretenden Lichtstrahl auf den lichtempfindlichen Bereich des elektrooptischen Sensorfeldes, das ein eindimensionales oder zweidimensionales Feld sein kann. Zweidimensionale elektrooptische Sensorfelder sind ebenso wie eindimensionale Felder im Handel erhältlich und werden bevorzugt verwendet, weil bei einer zweidimensionalen Vorrichtung eine relative Bewegung zwischen dem austretenden Lichtstrahl und dem elektrooptischen Sensorfeld vermieden werden kann.

Die Erfindung weist weiterhin den Vorteil auf, daß die Sensoreinheit aus billigen, stabilen und/oder robusten Elementen in einer kompakten Anordnung aufgebaut werden kann. Es sind zum Abtasten eines Fingerabdrucks und zur Umwandlung eines resultierenden Bildes in eine Reihe von elektrischen Signalen, welche die in einer herkömmlichen Bildverarbeitungseinheit verarbeitbare oder auswertbare Bildinformation enthalten, keine beweglichen Teile erforderlich.

0045914

-9-        VPA 80 P 8230

Bevorzugte Ausführungsformen der Erfindung werden anhand der Figuren in der nun folgenden Beschreibung näher erläutert. Von den Figuren zeigen

Figur 1 in schematischer Darstellung eine Ausführungsform einer Eingabesensoreinheit mit einer aus einem elastischen Polymer gebildeten Sensorplatte,

Figur 2 einen Ausschnitt aus der Sensorplatte, aus dem die Lichtverteilung in der Sensorplatte hervorgeht,

Figur 3 in Kombination mit der Figur 4 eine andere Ausführungsform einer Eingabesensoreinheit mit einem optischen Bandpaßfilter, und

Figur 6 und Figur 7 Ausführungsformen von Beleuchtungssystemen zur Erzeugung eines in die Sensorplatte einzukoppelnden abtastenden Lichtstrahls.

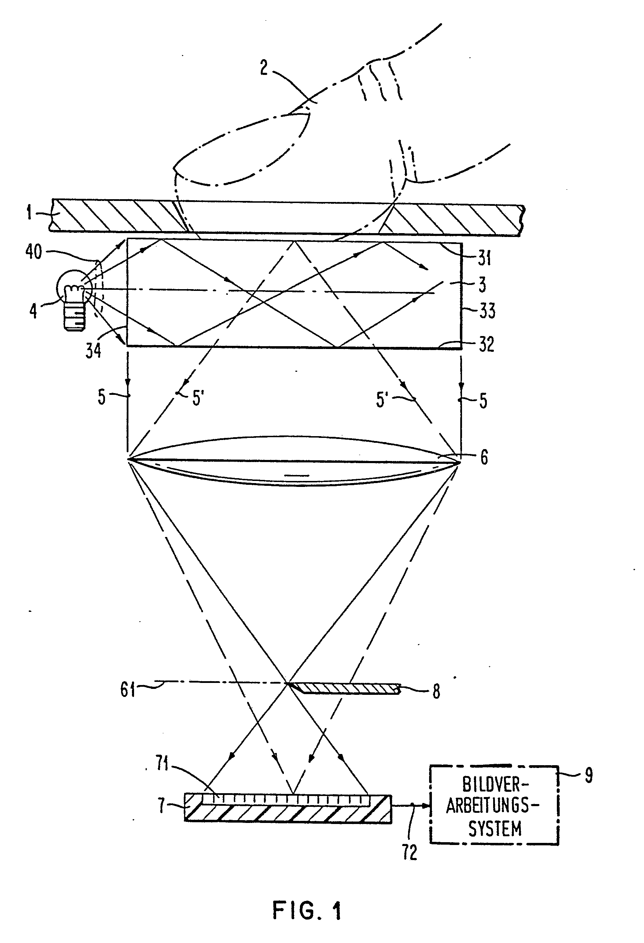

Der allgemeine Aufbau einer vorgeschlagenen Eingabesensoreinheit ist in der Figur 1 dargestellt. Sie
umfaßt ein Fingerbett 1, das eine zentrale Öffnung
zur Aufnahme eines Fingers 2 aufweist, der zur Veranschaulichung eines Betriebszustandes in strichpunktierten Linien 4 dargestellt ist.

Unter dem Fingerbett 1 und parallel dazu ist eine
Sensorplatte 3 angeordnet. Die Sensorplatte 3 ist
aus Gründen des besseren Verständnisses nicht
maßstabsgetreu dargestellt. Sie ist in Wirklichkeit
ein relativ dünner Streifen, der aus einem elastischen
Polymer besteht, und der parallel zueinander angeordnete planare Flächen 31 und 32 und senkrecht dazu
verlaufende Randflächen 33 und 34 aufweist. Die
Sensorplatte 3 besteht aus einem druckempfindlichen
elastischen Polymer. Die Elastizitätseigenschaften
der Sensorplatte sollten so ausgeprägt sein, daß ein
latentes, getreues topografisches Relief der Vertiefungen und Erhöhungen eines Fingers erhalten wird,
wenn der Finger 2 auf die erste planare Fläche 31
die dem Fingerbett 1 zugewandt ist, gedrückt wird.
Sensorplatten, welche diese Eigenschaften aufweisen,
können von einem im Handel erhältlischen Silikonelastomer, beispielsweise das von General Electric
hergestellte Silikonelastomer RTV 670 erhalten werden.

Neben der Sensorplatte 3 ist eine Lichtquelle 4 angeordnet, die einer Randfläche 34 der Platte gegenüberliegt und die einen abtastenden Lichtstrahl erzeugt,
der durch die gegenüberliegende Randfläche 34 in das
Innere der Sensorplatte 3 gerichtet ist. Wegen der
planparallelen Struktur der Sensorplatte 3 wird das in sie eingekoppelte Licht in ihr durch innere Reflexion verteilt. Solange die planparallele Struktur der Sensorplatte 3 nicht gestört ist, was bei der Abwesenheit eines Fingers der Fall ist, wird der abtastende Lichtstrahl total reflektiert und die Sensorplatte 3 strahlt kein Licht ab. Dieser ungestörte Zustand ändert sich immer dann, wenn die erste planare Fläche 31 mit einem Finger 2 beaufschlagt wird.

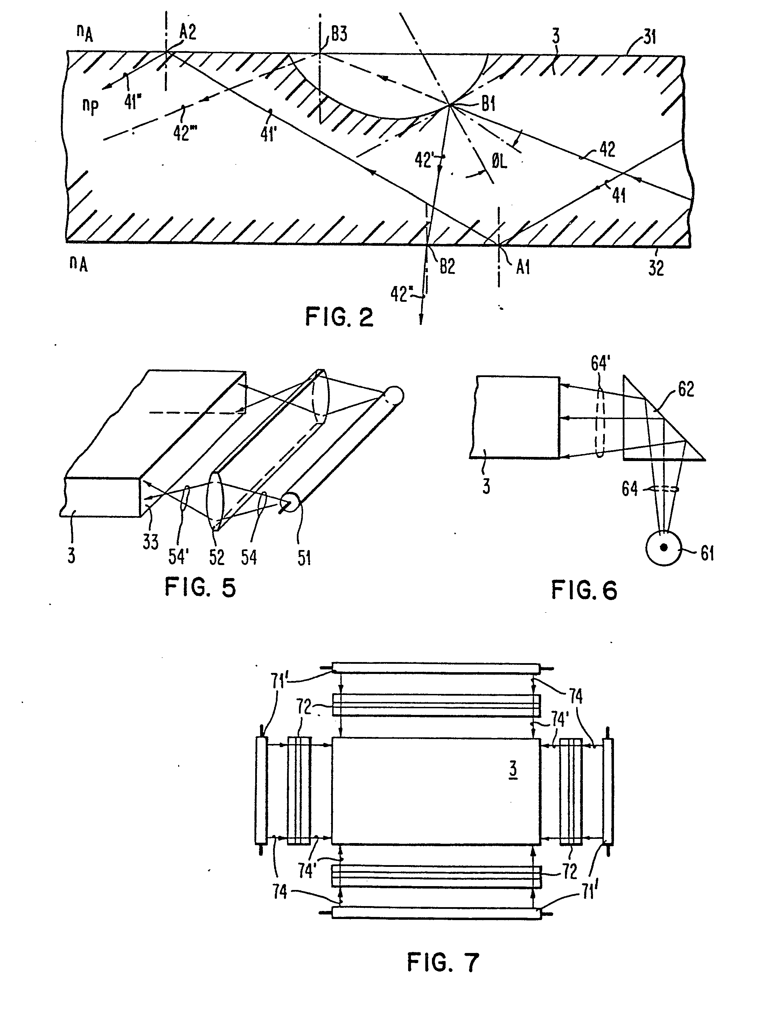

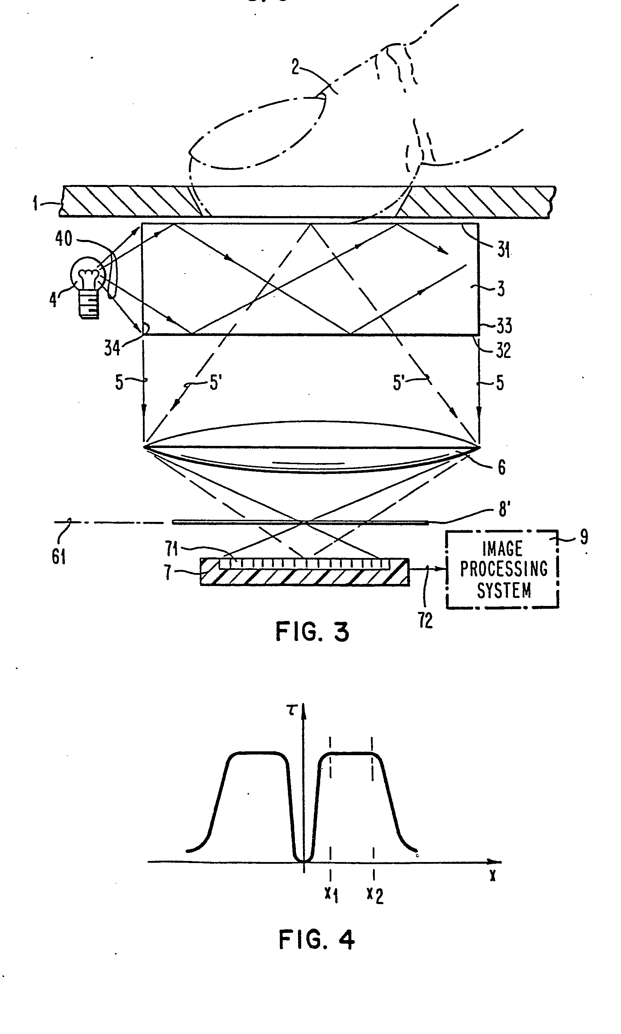

Die Prinzipien der Lichtverteilung des abtastenden Lichtstrahls 40 kann besser aus der in Figur 2 dargestellten detaillierteren Schemadarstellung der Sensorplatte 3 verstanden werden. Zwecks einer besseren Perspektive ist nur eine Vertiefung 35 in der ersten planaren Fläche 31 dargestellt. Diese Vertiefung 35 kann von einer Rippe eines Fingers herrühren.

Optisch betrachtet, bilden die Sensorplatte 3 und die umgebende Luft ein optisches System, welches aus drei Schichten zusammengesetzt ist. Die Sensorplatte 3, die zentrale Schicht, weist einen Brechungsindex $n_P$ auf. Beide planaren Flächen 31 und 32 liegen jeweils Luftschichten gegenüber, die einen Brechungsindex $n_A$ aufweisen. Nach dem Snelliusschen Gesetz existiert in einem derartigen System ein Totalreflexionswinkel, der durch

$$\sin \phi_L = n_A / n_P$$

bestimmt ist.

Aus Gründen einer besseren Perspektive sind nur zwei Lichtstrahlen und ihre reflektierten und abgelenkten Strahlen dargestellt. Ein erster Lichtstrahl 41 steht für alle Strahlen, die innen total reflektiert werden. Dieser erste Strahl 41 trifft die zweite planare Fläche 32 in einem Punkt A1 unter einem Einfallswinkel, der größer als der Totalreflexions- winkel $\phi_L$ ist, und dieser Strahl wird total reflektiert und geht in den reflektierten Strahl 41' über. Dieser Strahl 41' trifft seinerseits die erste planare Fläche 31 in einem Punkt A2 und wird dort wieder reflektiert, so daß er in den Strahl 41'' übergeht. Da alle Strahlen dieser Gruppe innerhalb der Sensorplatte 3 ähnlich intern reflektiert werden, wird kein Licht in die umgebenden Bereiche abgestrahlt, d.h. die planaren Flächen 31 und 32 sind total dunkel, wenn sie senkrecht betrachtet werden.

Dieser Zustand ändert sich immer dann, wenn eine planare Fläche gestört wird, beispielsweise durch die Vertiefung 35. Der Strahl 42, der die Gruppe von Strahlen repräsentiert, welche eine lokal gestörte Fläche treffen, geht durch interne Reflexion im Punkt B1 in einen reflektierten Strahl 42' über, weil er die tangentiale Ebene - wie schematisch dargestellt - unter einem Winkel trifft, der größer ist als der begrenzende Totalreflexionswinkel $\phi_L$. Der reflektierte Strahl 42', der die zweite planare Fläche 32 im Punkt B2 unter einem Einfallswinkel trifft, der kleiner ist als der begrenzende Totalreflexionswinkel $\phi_L$, wird dort gebrochen und geht in den Strahl 42'' über. Ohne eine lokale Verformung der ersten Fläche 31 würde der zweite Strahl 42 diese Fläche in einem Punkt B3 treffen, intern reflektiert werden und in einen Strahl 42''' übergehen. Dies zeigt, daß eine Verformung der horizontalen Ebene der ersten planaren Fläche 31 bei Gegenwart eines Fingers in Lichtstrahlen resultiert, die durch die zweite planare Fläche
32 der Sensorplatte 3 strahlen.

Es dürfte verständlich sein, daß entsprechende Ergebnisse erzielt werden können, wenn der Brechungsindex
$n_p$ der Sensorplatte 3 in Abhängigkeit von der Durchdringungstiefe des Lichts in einer zu den ebenen
Flächen 31 und 32 senkrechten Richtung variiert.

Ähnlich zu der Ausführungsform nach Figur 2 kann
eine entsprechende optische Ausführungsform aus
mehreren mittleren Schichten, deren Brechungsindizes
in Abhängigkeit vom Abstand von der ersten planaren
Fläche zunimmt, zeigen, daß Rippen oder Erhöhungen
eines Fingerabdrucks deutlicher oder charakteristischer
festgestellt werden können.

Der Anordnung der in Figur 1 dargestellten Eingabesensoreinheit ist zu entnehmen, daß die Sensorplatte
3 durch ihre zweite planare Fläche 32 Licht emittiert,
das aus zwei Lichtstrahlen zusammengesetzt ist,
einem aus Strahlen 5 bestehenden Lichtstrahl, der
einen sogenannten Parallelanteil bildet und einem
aus Strahlen 5' zusammengesetzten modulierten Lichtstrahl, der die benutzte Fingerabdruckinformation
enthält.

Beide Lichtstrahlen werden in einem optischen Linsensystem 6 auf der Seite der zweiten planaren Fläche 32
der Sensorplatte 3 gebrochen. Zur Reproduzierung von
aus dem Fingerabdruck in einem fotoempfindlichen Bereich 71 eines elektrooptischen Abtastfeldes 7
resultierenden Phasenstrukturen ist das zwischen
der Sensorplatte 3 und diesem elektrooptischen Abtastfeld angeordneten Linsensystem so gewählt, daß der aus den Strahlen 5' bestehende modulierte Lichtstrahl auf die optisch empfindliche Fläche 71 projiziert wird. Da der aus den Strahlen 5 bestehende direkte Lichtstrahl auf Verformungen der Phasenstrukturen in der Brennebene des optischen Linsensystems 6 enthält, ist zum Ausblenden des in dieser Ebene fokussierten direkten Lichtstrahls ein optisches Filter vorgesehen. In der Ausführungsform nach Figur 1 ist das Filter als sogenannte Schneide 8 ausgebildet, die ein konventionelles Mittel zum Ausblenden spezieller Teile aus Lichtstrahlen für verschiedene Objektive darstellt. Ein Beispiel dafür ist in "Optics" von Hecht und Zajak, herausgegeben von Addison-Wesely Publishing Comp., S. 481, beschrieben. Die Schneide 8 in der hier vorgeschlagenen Anordnung ist in der Brennebene 61 des optischen Linsensystems 6 so einjustiert, daß sie den auf diese Ebene fokussierten Lichtstrahl nicht durchläßt.

Das auf diese Weise dem fotoempfindlichen Bereich 71 des elektrooptischen Sensorfeldes 7 dargebotene optische Bild von Phasenstrukturen wird in elektrische Signale umgewandelt, welche die vollständige Information der Phasenstruktur enthalten. Für diese Umwandlung kann jedes herkömmliche elektrooptische Abtastfeld benutzt werden. Im Siemens Forschungs- und Entwicklungsberichte, Vol. 8, Nr. 5, Springer-Verlag, 1979, S. 268 - 271 ist beschrieben, daß derartige Felder aus einer regelmäßigen Anordnung von fotoempfindlichen Zellen besteht, die in einer eine Abtastzeile bildenden Reihe oder für parallele Anwendungen auf einem Schild angeordnet sind. Zum Aufzeichnen eines Bildes durch eine Abtastzeile muß das aufzuzeichnende Feld oder Objekt so bewegt werden, daß ein zeilenweises Abtasten

0045914

-15- VPA 80 P 8230

ermöglicht ist. Diese mechanische Bewegung wird bei Verwendung eines zweidimensionalen Abtastfeldes vermieden.

Heutige Festkörper- Bildsensorfelder sind im Handel erhältlich, beispielsweise das zweidimensionale selbstabtastende optische Sensorfeld Reticon RA 100 x 100, welches symmetrisch in einer 100 x 100-Matrix angeordnete diskrete Fotodioden aufweist. Dieses Feld wird von EG. & G. Reticon, Sunnyvale, Kalifornien, USA, verkauft.

Elektrische Signale 72, welche die Information des optischen Bildes darstellen, werden in ein Bildverarbeitungssystem 9 übertragen, welches mit einer strichpunktierten Linie schematisch dargestellt ist, und wodurch angedeutet werden soll, daß die weitere Verarbeitung der elektrischen Signale kein Bestandteil der vorliegenden Erfindung ist. Gemäß den eingangs angegebenen Ausführungen zum Stand der Technik sind Verfahren und Anordnungen für die Bildverarbeitung bekannt. Eine eingehendere Beschreibung der Signalverarbeitung zur Verifizierung eines Fingerabdrucks erscheint deshalb nicht als erforderlich.

In der Beschreibung der bevorzugten Ausführungsform ist darauf hingewiesen, daß zwischen dem optischen Linsensystem 6 und dem elektrooptischen Sensorfeld 7 angeordnete optische Filter zur Verbesserung der Qualität des dem fotoempfindlichen Bereich des elektrooptischen Sensorfeldes 7 dargebotenen Bildes sehr gebräuchlich sind. Da eine Schneide, obwohl sie zum Ausblenden des direkten Lichtstrahls sehr gebräuchlich ist, selbst eine gewisse Störung im Lichtweg verursacht, können andere optische Filter sogar mehr bevorzugt werden.

Eine zweite Ausführungsform der Erfindung, die eine andere Art eines optischen Filters enthält, ist in der Figur 3 dargestellt. Vergleichbare Elemente beider Ausführungsformen weisen identische Bezugzeichen auf und sind identisch dargestellt, wodurch die folgende Beschreibung auf in dieser zweiten Ausführungsform benutzte optische Filter beschränkt ist. Es ist ein konventionelles optisches Bandpaßfilter 8'. Es ist in dem Brennpunkt des optischen Linsensystems 6 in der Brennebene 61 zentriert. Die Zusammensetzung des optischen Bandpaßfilters kann aus der unter der Figur 3 gezeigten Figur 4 erhalten werden. Diese Figur 4 zeigt ein Diagramm, welches den Durchlaß-koeffizienten $\tau$ in Abhängigkeit vom Abstand x vom Mittelpunkt darstellt. Die Durchlaßcharakteristik ist radial symmetrisch. Der Durchlaßkoeffizient $\tau$ ist im Mittelpunkt O, steigt mit wachsendem Abstand x von diesem Mittelpunkt bis auf einen Maximalwert an, der in einem kurzen Bereich zwischen den Abständen $x_1$ und $x_2$ beibehalten wird, und nimmt dann mit weiter wachsendem Abstand vom Mittelpunkt scharf ab. Dies ist der Bereich, in dem der aus den Strahlen 5' bestehende modulierte Lichtstrahl auf das optische Bandpaßfilter auftrifft.

Neben der optischen Filterung des dem fotoempfindlichen Bereich des elektrooptischen Sensorfeldes 7 dargebotenen Bildes liegt ein anderer wesentlicher Aspekt der Erfindung darin, wie der abtastende Lichtstrahl 40 erzeugt und die Sensorplatte 3 eingekoppelt wird. Anordnungen zum Einkoppeln des abtastenden Lichtstrahls 40 in die Sensorplatte 3 sind schematisch in den Figuren 5 bis 7 dargestellt.

Figur 5 zeigt schematisch und in isometrischer Darstellung eine Teilansicht einer Sensorplatte 3 mit einer Stirnfläche 33, der eine Zylinderlinse 52 gegenüberliegt, die so angeordnet ist, daß ihre Längsachse parallel zur Stirnfläche 33 verläuft. Auf der von der Stirnfläche 33 abgewandten Seite der Zylinderlinse 52 ist eine röhrenförmige Lichtquelle 51 vorgesehen. Das von dieser Quelle imitierte Licht wird von der Zylinderlinse gebrochen und der Stirnfläche 33 der Sensorplatte 3 zugeführt. Das Licht dringt in die Sensorplatte 3 ein und bleibt wie oben beschrieben darin gefangen. Der Grund für die optische Koppelanordnung gemäß Figur 5 liegt darin, daß eine geeignet gestreute Beleuchtung in Richtung der Längsachse der Stirnfläche 33 erhalten wird. Wie es durch die gebrochenen Strahlen 54, die einen abtastenden Lichtstrahl enthalten, gezeigt ist, kann die Verteilung des längs einer senkrecht auf der Längsachse der Randfläche 33 stehenden Achse eindringenden Lichts geeignet diffus gehalten werden.

Figur 6 zeigt im Vergleich zur Anordnung gemäß Figur 5 anstelle einer Zylinderlinse ein Totalreflexionsprisma 62, das zwischen der Randfläche 33 der Sensorplatte 3 und einer Lichtquelle 61 angeordnet ist. Da die Anordnung des optischen Koppelsystems derart ausgebildet ist, daß die Lichtquelle 61 Lichtstrahlen 64 unter verschiedenen Einfallswinkeln imittiert, die kleiner als der begrenzende Totalreflexionswinkel sind, werden die entsprechenden reflektierten Strahlen 64' vom Prisma 62 unter verschiedenen Winkeln ausgesandt und dringen durch die Randfläche 33 in die Sensorplatte 3 ein.

Das optische Koppelsystem gemäß Figur 6 ermöglicht
die Anordnung der Lichtquelle 61 seitlich und unterhalb oder oberhalb der Sensorplatte 3. Im Vergleich
zur Anordnung gemäß Figur 5 wird dadurch eine räumlich
kompaktere Bauweise erreicht.

Eine der Haupteigenschaften der optischen Anordnung
zum Einkoppeln des abtastenden Lichtstrahls in die
Sensorplatte 3 liegt, wie schon angedeutet, darin,
in der Sensorplatte eine diffuse Lichtverteilung
zu erreichen. Die in Figur 5 dargestellte Anordnung
ist so ausgebildet, daß zur diffusen Beleuchtung einer
Randfläche 33 eine dieser gegenüberliegende lineare
Lichtquelle und eine Zylinderlinse benutzt werden.
Ein noch besseres Ergebnis wird erhalten, wenn durch
alle vier Randfläche Licht in die Sensorplatte 3 eingekoppelt wird. Eine derartige Anordnung ist in der
Figur 7 in Draufsicht und schematisch dargestellt.

Diese symmetrische Anordnung röhrenförmiger Lichtquellen 71' und zylindrischer Linsen 72 garantiert,
daß es keine bevorzugte Richtung zum Einkoppeln des
abtastenden Lichtstrahls in die Sensorplatte 3 gibt.
In diesem Fall wird Licht von den röhrenförmigen
Lichtquellen 71' ausgesandt, von denen jede einer
Randfläche der Sensorplatte 3 gegenüberliegt. Vier
Lichtstrahlen sind durch Strahlen 74 dargestellt,
welche von einer zugeordneten der zylindrischen
Linsen 72 durch Brechung in einen gebrochenen Lichtstrahl 74' umgewandelt werden. Das eindringende Licht
wird innerhalb der Sensorplatte durch Totalreflexion
verteilt und beleuchtet die druckempfindliche planare
Fläche ohne Vorzugsrichtung, wodurch unverfälschte
oder verzerrte Abtastresultate erhalten werden.

13 Patentansprüche

7 Figuren

Patentansprüche

1. Eingabesensoreinheit für ein System zum Überprüfen von Fingerabdrücken, g e k e n n z e i c h n e t   d u r c h

a) ein Fingerbett (1) mit einer Öffnung zur Aufnahme eines Fingers (2), dessen Abdruck zu überprüfen ist,

b) eine Sensorplatte (3), die aus einem transparenten elastischen Material besteht und die eine erste (31) und eine zweite große planare Fläche (32) und Randflächen (33, 34) aufweist, wobei die erste planare Fläche (31) neben dem Fingerbett (1) angeordnet ist, parallel dazu verläuft, sich über die Öffnung (1) des Fingerbettes erstreckt und so elastisch ist, daß sie ein latentes topografisches Relief eines Fingermusters ausbildet, wenn ein Finger auf das Fingerbett gelegt wird,

c) eine Beleuchtungseinrichtung (51, 52; 61, 62; 71, 72) zum Erzeugen eines durch wenigstens eine Randfläche (33) in die Sensorplatte (3) eindringenden abtastenden Lichtstrahls (54'; 64'; 74'), mit dem ein aus der zweiten planaren Fläche (32) austretender Lichtstrahl (42'') erzeugbar ist,

d) ein elektrooptisches Sensorfeld (7) mit einem fotoempfindlichen Bereich (71), das dem austretenden Lichtstrahl (42'') ausgesetzt ist, und

e) eine zwischen der Sensorplatte (3) und dem elektrooptischen Sensorfeld (7) angeordnete optische Einrichtung (6) zum Fokussieren des modulierten austretenden Lichtstrahls (5, 5') auf den fotoemfpindlichen Bereich (71),

wodurch bei Abwesenheit eines Fingers der abtastende Lichtstrahl im Inneren der Sensorplatte (3) an den planaren Flächen total reflektiert wird, während in Gegenwart des Fingers (2) die geriffelte erste planare Fläche (31) den entsprechenden modulierten austretenden Lichtstrahl so reflektiert, daß er aus der zweiten planaren Fläche (32) austritt und auf den fotoempfindlichen Bereich (71) des elektrooptischen Sensorfeldes (7) trifft.

2. Eingabesensoreinheit nach Anspruch 1, d a d u r c h   g e k e n n z e i c h n e t , daß die Sensorplatte (3) ein elastisches Polymer aufweist.

3. Eingabesensoreinheit nach Anspruch 2, d a d u r c h   g e k e n n z e i c h n e t , daß die Sensorplatte (3) ein elastisches Polymer aufweist, welches einen in Abhängigkeit von der Eindringtiefe senkrecht zu den großen planaren Flächen (31, 32) der Sensorplatte (3) abnehmenden Brechungsindex aufweist.

4. Eingabesensoreinheit nach Anspruch 3, d a d u r c h   g e k e n n z e i c h n e t , daß der Bereich variierenden Brechungsindexes auf die Tiefe des Reliefs des Fingermusters begrenzt ist.

5. Eingabesensoreinheit nach einem der vorhergehenden Ansprüche, d a d u r c h   g e k e n n z e i c h - n e t , daß die Beleuchtungseinrichtung eine kohärente Lichtquelle aufweist.

6. Eingabesensoreinheit nach einem der Ansprüche 1 bis 4, d a d u r c h   g e k e n n z e i c h - n e t , daß die Beleuchtungseinrichtung (51, 52; 61, 62; 71, 72) eine inkohärente Lichtquelle (51; 61; 71) aufweist.

7. Eingabesensoreinheit nach Anspruch 6, d a d u r c h   g e k e n n z e i c h n e t , daß die inkohärente Lichtquelle (51; 61; 71) eine röhrenförmige Lichtquelle aufweist, deren Längsachse parallel zur Längsachse einer zugeordneten Randfläche (33) der Sensorplatte (3) angeordnet ist.

8. Eingabesensoreinheit nach Anspruch 7, d a d u r c h   g e k e n n z e i c h n e t , daß die Beleuchungseinrichtung (51, 52; 71', 72) eine zwischen der röhrenförmigen Lichtquelle und der zugeordneten Randfläche (33) angeordnete Zylinderlinse (52; 72) aufweist.

9. Eingabesensoreinheit nach Anspruch 7, d a d u r c h   g e k e n n z e i c h n e t , daß die Beleuchtungseinrichtung (61, 62) ein zwischen der röhrenförmigen Lichtquelle und der zugeordneten Randfläche (33) angeordnetes optisches Koppelprisma (62) aufweist.

10. Eingabesensoreinheit nach einem der Ansprüche 6 bis 9, d a d u r c h   g e k e n n z e i c h n e t , daß mehrere röhrenförmige Lichtquellen (71') vorgesehen sind, von denen jede mit ihrer Längsachse parallel zur Längsachse einer zugeordneten der mehreren Randflächen der Sensorplatte (3) angeordnet ist.

11. Eingabesensoreinheit nach einem der vorhergehenden Ansprüche, d a d u r c h   g e k e n n z e i c h - n e t , daß die optische Einrichtung (6, 8; 6, 8')
a') eine optische Linse (6) aufweist, welche der zweiten planaren Fläche (32) der Sensorplatte (3) gegenüberliegt, und
b') ein optisches Filter (8; 8') aufweist, welches zwischen der optischen Linse (6) und dem elektrooptischen Sensorfeld angeordnet ist und zur räumlichen Ausfilterung des senkrecht aus der zweiten planaren Fläche (32) austretenden und damit im austretenden Lichtstrahl (5, 5') enthaltenen direkten Lichts dient.

12. Eingabesensoreinheit nach Anspruch 11, d a - d u r c h  g e k e n n z e i c h n e t , daß das optische Filter (8) eine Schneide aufweist, die in einer Brennebene der optischen Linse (6) angeordnet ist, und die in den modulierten austretenden Lichtstrahl (5, 5') bis zu dessen optischer Achse ragt.

13. Eingabesensoreinheit nach Anspruch 11, d a - d u r c h  g e k e n n z e i c h n e t , daß das optische Filter (8') ein optisches Bandpaßfilter aufweist, welches in einer Brennebene der optischen Linse (6) angeordnet ist und verschiedene Durchlaßkoeffizienten aufweist, derart, daß mit zunehmendem radialem Abstand von der optischen Achse des modulierten Lichtstrahls (5, 5') der Transmissionskoeffizient von Null auf einen maximalen Wert zunimmt, der in einem kleinen Bereich unverändert bleibt und daß der Transmissionskoeffizient danach sehr rasch abnimmt.

FIG. 1

**FIG. 2**

**FIG. 5**

**FIG. 6**

**FIG. 7**

FIG. 3

FIG. 4

Europäisches
Patentamt

**EUROPÄISCHER RECHERCHENBERICHT**

| | EINSCHLÄGIGE DOKUMENTE | | KLASSIFIKATION DER ANMELDUNG (Int. Cl ³) |
|---|---|---|---|
| Kategorie | Kennzeichnung des Dokuments mit Angabe, soweit erforderlich, der maßgeblichen Teile | betrifft Anspruch | |
| D | US - A - 4 120 585 (V.A. DE PALMA)<br>* Zusammenfassung; Abbildungen * | 1,2,6 | A 61 B 5/10 |
| | IBM TECHNICAL DISCLOSURE BULLETIN, Band 8, Nr. 3, August 1965, Seiten 435-436<br>New York, U.S.A.<br>C.H. CLAASSEN et al.: "Fingertip orienting and ridge viewing apparatus"<br>* Insgesamt * | 1,6 | |
| | US - A - 3 619 060 (J.E. JOHNSON)<br>* Zusammenfassung; Abbildung 2; Spalte 3, Zeile 52 - Spalte 4, Zeile 19 * | 1,6,11 | RECHERCHIERTE SACHGEBIETE (Int. Cl ³)<br><br>A 61 B 5/10<br>G 07 C 9/00<br>11/00 |
| A | US - A - 3 865 488 (E.H. DEL RIO)<br>* Zusammenfassung; Abbildungen * | 1,6 | |
| A | US - A - 3 407 715 (C.W. McCUTCHEN)<br>* Zusammenfassung; Abbildung 3; Spalte 3, Zeilen 34-41 * | 1,6,9 | KATEGORIE DER GENANNTEN DOKUMENTE<br><br>X: von besonderer Bedeutung<br>A: technologischer Hintergrund<br>O: nichtschriftliche Offenbarung<br>P: Zwischenliteratur<br>T: der Erfindung zugrunde liegende Theorien oder Grundsätze<br>E: kollidierende Anmeldung<br>D: in der Anmeldung angeführtes Dokument<br>L: aus andern Gründen angeführtes Dokument<br>&: Mitglied der gleichen Patentfamilie, übereinstimmendes Dokument |

Der vorliegende Recherchenbericht wurde für alle Patentansprüche erstellt.

| Recherchenort | Abschlußdatum der Recherche | Prüfer |
|---|---|---|
| Den Haag | 19-11-1981 | DAVID |

EPA form 1503.1  06.78